# EUROPEAN PATENT SPECIFICATION

(11) **EP 3 893 977 B1**
(45) Date of publication and mention of the grant of the patent: **07.08.2024**
(21) Application number: 19824404.8
(22) Date of filing: 09.12.2019
(51) Int. Cl.: A61M 21/02, A63H 3/00

(54) **SLEEP-INDUCING TOY**
SCHLAFINDUZIERENDES SPIELZEUG
JOUET INDUISANT LE SOMMEIL

(30) Priority: 10.12.2018 NL 2022159
(43) Date of publication of application: 20.10.2021
(73) Proprietor: O'Poppy B.V., 1014ZE Amsterdam (NL)
(72) Inventor: GMELIG, Lucas Georg Balthasar, 1014 ZE Amsterdam (NL); BLOKKER, Lotta, 1014 ZE Amsterdam (NL)
(74) Representative: HGF
(86) International application number: PCT/NL2019/050816
(87) International publication number: WO 2020/122710

(56) References cited:
- EP-A2- 0 218 182
- WO-A2-2015/118302
- GB-A- 2 471 333
- US-A1- 2002 035 995

## Description

The present invention relates to sleep-inducing toys, more particular to sleep-inducing toys comprising a built-in body that produces a mechanical vibration, in particular for inducing and/or maintaining sleep in babies and toddlers as defined in the appended claims.

Some babies and toddlers have difficulties falling asleep, or to stay asleep for longer periods of time. Instead, they often wake up numerous times during the night, resulting in fatigue of children and parents or carers alike, due to sleepless nights.

While there are many ways disclosed to comfort the babies and toddlers with devices that mimic for example the sound of the womb that a foetus observes, or easeful music, or breathing sounds, most of these devices fail to induce the desired effect. Furthermore, while the sounds of a vacuum cleaners or tumble dryers are noted to sometimes helps babies to fall asleep, or the movement of a pram, these are rather unwieldy devices, and in particular are not suited for travel purposes. On the other hand, there are many toys or dolls on the market that have a comforting function due to touch and feel, but are not actively inducing and maintaining sleep.

An object in the form of a toy, which comprises an apparatus which can record for example the breath sound or artificially synthesized voices has been disclosed for instance in JP-A-3192253, whereby it is claimed that users may fall asleep more easily when employing this device.

In EP0218182 a child pacifying device is described, comprising a soft doll body with a pulsating device disposed in the body. The pulsating device mimics the heart beat by alternating between a strong pulse and a weaker pulse.

An objective of this invention is to provide sleep inducing toys that improve the comfort in use and provide convenience for sleeping of a user.

It is a further object of this invention to provide a sleep-inducing toy that makes a sound and vibrates at the same time, to create an additionally comforting effect with regards to the sleeping efficiency.

Another object of this invention is to provide an apparatus that produces a sound and vibration at certain frequencies that are optimized for giving a pleasant and comforting experience.

A still further object of this invention is to provide an apparatus that has an optimised shaped for transferring vibration via a medium to a user.

It is a still further object of this invention to provide a toy of the above-described type which is relatively simple in construction and can be economically manufactured.

These and other features and characteristics of sleep-inducing toys according to the present invention will be apparent from the following description and accompanying illustration of typical embodiments thereof.

In a first aspect, the present invention relates to a vibration module for a sleep-inducing toy, the module comprising a vibration apparatus configured to produce a mechanical vibration generating at least two mechanical waves of different frequencies for transferring via a medium to a user, wherein the frequencies produce both a sound and a vibration; and wherein the vibration module generates a mechanical oscillation wherein the mechanical vibration generates at least one frequency in the range of from 40 up to 45 Hz, and at least one frequency in the audible range, in the range of from 60 up to 65 Hz.

Applicants found via intensive testing that the combination of sound and vibration worked best for comforting a user.

Advantageously, the apparatus produces a mechanical vibration generating least 3 different wave frequencies. Advantageously, the mechanical vibration induces at least an 3 different wave frequencies below 160 Hz, more preferably between 20 and 120 Hz.

Advantageously, the mechanical vibration induces at least an undertone in the range of from 100 up to 160 Hz, and an overtone in the range of from 200 up to 300 Hz.

Advantageously, the mechanical vibration generates a further overtone in the range of from 400 up to 500 Hz.

Advantageously, the mechanical vibration comprises waves of 3 frequencies, which frequencies are in the range of from 120 to 140 Hz, 250 up to 270 Hz and 450 up to 470 Hz, respectively.

In a further embodiment, the mechanical vibration comprises waves of at least 2 frequencies, up to 6 frequencies, more preferably 2-5 frequencies, which frequencies are in the range of from 40 to 45 Hz, 52 up to 58 Hz, 60 up to 65 Hz, 70 up to 75 Hz and 90 up to 95 Hz, respectively.

In an even further embodiment, the mechanical vibration starts with the higher frequencies, and after a certain time interval, it switches to a lower frequency for a certain time interval, and in an even further preferred embodiment, the frequency is further reduced for a certain time interval. Time intervals may vary between 2 minutes up to 60 minutes, and in certain circumstances, even time intervals of 10 seconds up to 2 minutes can be used. Also combinations of time intervals can be used, e.g. 5 minutes at the highest frequency levels, followed by for example 40 minutes during a lower frequency, followed by 1 minute at an even lower frequency.

We have tested an apparatus that comprised a vibrational tactile effect with frequencies of 130 Hz, 260 Hz and 460 Hz, induced via mechanical oscillation, and with these frequencies an optimum of comfort was achieved.

We have furthermore tested an apparatus that comprised a vibrational tactile effect with frequencies of 92 Hz, 62 Hz and 41 Hz, induced via mechanical oscillation, whereby the frequency of 92 Hz was applied for 5 minutes, the frequency of 62 Hz was applied for 35 minutes and that was furthermore reduces to 41 Hz for 5 minutes, and then even further reduced for 15 sec, before it stopped vibrating. This gave good comfort for the child to remain asleep during the night.

That comfort was achieved both via an optimum in transmittal of a vibration via a medium to the user, and the observation of the sounds via the ears of the user.

The medium can be any medium suitable for transferring vibration to a user. Preferably, the medium can be a sleeping pillow or a matrass, preferably of sufficiently high density to transfer the vibration.

Advantageously, the sleep-inducing toy has a size of from 15 centimetres up to 40 centimetres long and a width of from 5 up to 20 centimetres. Preferably, the size of the toy is being designed for the size suitable for the user. For example, for a baby, a smaller toy may be more suitable and for a toddler or a child, a bigger size of the toy may be more suited.

Advantageously, the toy comprises an outer shell or sleeve that is made from a soft fabric, preferably washable, such as from cotton or polyester fabric. Preferably, the sleeve is designed such that it gives the toy the aspect of a "friendly" fantasy animal.

The vibration module can be advantageously used in a toy which is both a thing to play and cuddle with and a sleep aid. A child may play with the toy, and familiarise itself with it, whereby the toy is particularly effective as a sleep aid due to the comfort provided to a child by a familiar toy.

The sleep-inducing toy comprises a vibration and sound wave generator, preferably a vibration motor, more preferably a bar-type vibration motor.

In the latter case, an off-centred weight is attached to an electric motor's rotational shaft that produces a centrifugal force while rotating. This unbalanced force displaces the motor, resulting in a vibration. Since the motor is affixed in the apparatus, the vibration is thus transferred to the toy.

Preferably, rotor size, off-set distance and motor weight are chosen such that the motor generates a primary vibration, as well as overtones that are transferred as audible sounds.

The vibration module preferably further comprises a controller, wherein the vibration module is operatively connected to the controller

The vibration module preferably further comprising an electric power source, preferably a rechargeable electric battery, optionally comprising an induction charger.

The vibration module preferably generates a mechanical oscillation, which may advantageously be induced by a bar-type vibration motor. Preferably, the vibration apparatus comprises an electric motor comprising a drive axle, and a rotor operably connected to the drive axle.

The weight of the mechanical oscillator is being optimised based on the force needed to transfer the vibration through the sleeve and medium to the user. It depends on both the size of the motor, which might differ per model of the sleep-inducing toy, the design of the sleeve and the user type. The rotating frequency is in the range of the undertone from 100 up to 160 Hz. The weight is being placed off-centre, preferably in the range of from 120 to 240 degrees off centre, more preferably in the range of from 150 to 210 degrees of centre, even more preferably of from 170 up to 190 degrees of centre. In the most preferred embodiment, the weight is being placed 180 degrees off-centre (half a circle).

Advantageously, the apparatus comprises a switch, or button, for turning the controller, and hence the vibration on and off. Advantageously the controller may also be operated remotely, using e.g. Bluetooth connectivity, or it may be programmed to operate for a certain period of time.

Advantageously, the vibration module further comprises a housing affixed to the vibration apparatus for transferring at least part of the vibrations to a sleeping pillow or a matrass in contact with the housing. Preferably, the housing comprises a bell-shaped upper body and a flat basis, to enhance the transfer of the vibrations to the medium. The advantage of such design is that transfer of the vibration to a pillow or matrass is better than with other shape, as the module follows the form of the sleeve. It furthermore provides the opportunity to maintain a soft and comfortable upper side of the sleeve while allowing the vibration to be transferred via the bottom side. The upper side is designed to be in contact with the user through the sleeve. The bottom side is being designed to have a maximum contact surface with the medium through the sleeve.

The housing may be made of any suitable material, such as polymeric materials usually used for electric apparatus, e.g. ABS, HDPP, Nylon, or other suitable polymers.

The present invention also relates to a sleep-inducing toy comprising the vibration module as described above is being used for comforting a person. Advantageously, this person is a child which is comforted to fall asleep. More advantageously, it is a baby or a toddler.

Embodiments of the invention are further described hereinafter with reference to the accompanying drawings, wherein like letters and numerals refer to like parts, wherein the figures are approximately to scale, and wherein:
Fig. 1 illustrates an example of a toy, showing the top view, bottom view and side view;
Fig. 2 illustrates an example of the front view of a vibration module;
Fig. 3 illustrates an example of the back view of a vibration module;
Fig. 4 illustrates an example of the top view of a vibrating module;
Fig. 5 illustrates an example of the shape of the vibrating module;
Fig. 6 illustrates an example of a block diagram of the inside of the vibrating module.

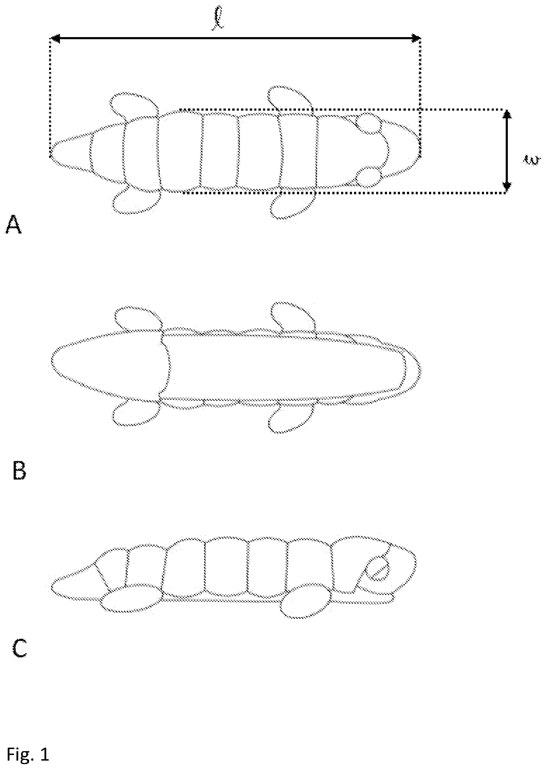

Figure 1 illustrates an example of a toy, showing the top view (A), the bottom view (B) and side view (C). The length of the toy is defined as (1) from the front to the back of the toy, and is preferably from 15 centimetres up to 40 centimetres. Preferably the baby version of the toy is smaller than the toddler version of the toy, the baby version being between 15 and 25 centimetres long and the toddler version between 25 and 40 centimetres long. The width of the toy is defined is (ω), and is preferably of from 5 centimetres up to 20 centimetres, more preferably of from 7 centimetres up to 12 centimetres. The smaller sizes enhance ease of transport of the toy in a smaller sized bag.

As can be seen in the sideview C, the bottom side of the toy is preferably flat to have a better transfer of the vibrations from the apparatus via a matrass of pillow or another medium to the user. Advantageously, the toy is designed as such that it is clear from the shape what the bottom of the toy is and that the apparatus is placed in the optimal configuration.

Advantageously, the outside or the sleeve of the toy, enveloping the housing, is made of a thin, durable and strong fabric that can be removed and washed. The fabric comprises preferably cotton. Advantageously, the toy comprises a soft and tactile upper side for more comfort of the user. Advantageously, the top side of the toy comprises a soft and deformable material between the vibration module and the outside of the toy, e.g. fibrefill, flake filling, polyether materials, polystyrene materials, cotton materials, foam rubber, and/or polystyrene spheres. Advantageously, there is no further material present between the bottom side of the vibration module and the outside of the toy, for increased transfer of vibrations to a medium, for example a mattress.

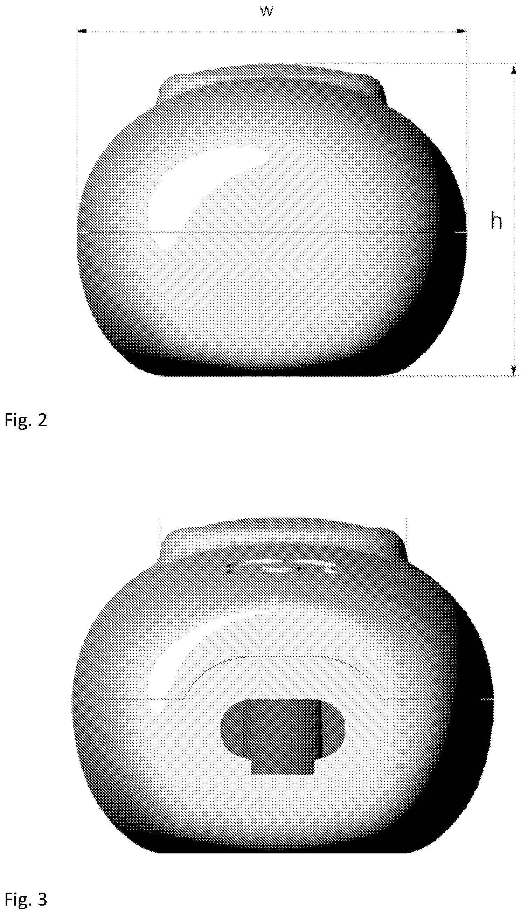

Figure 2 illustrates the front view of a vibration module, with w the width and h the height of the vibrating module.
Fig. 3 illustrates an example of the back view of a vibration module, with a charging access point clearly visible;

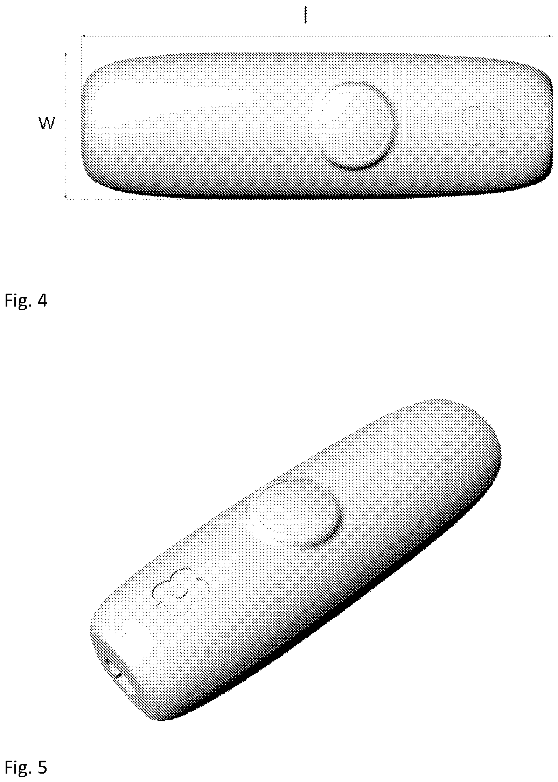

Fig. 4 illustrates an example of the top view of a vibrating module, with w the width and I the length of the vibrating module;
Fig. 5 illustrates an example of the shape of the vibrating module, wherein the shape of the edges is clearly visible to be rounded. It shows the housing comprising a bell-shaped upper body and a flat basis, to enhance the transfer of the vibrations to the medium

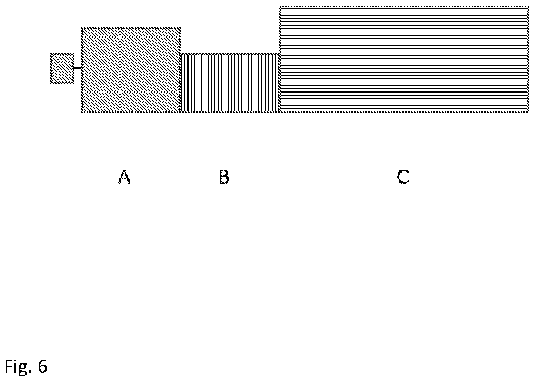

Fig. 6 illustrates a block scheme of the vibrating module.

The vibrating module is typically smaller than the toy. It advantageously comprises a switch (1) for turning it on and off, in this example it is a flat switch. The bottom (2) of the apparatus is preferably flat, which is advantageous for transfer of the vibrations. Advantageously, the vibration is experienced both mechanically and in an auditive way, as sound, preferably coming from one source. The mechanical vibration leads to a tactile experience via the medium to the body of the user, while the sound being produced by the mechanical vibration travels both through the medium to the body of the user and through the air to the ears of the user.

The edges (see figures) of the apparatus are preferably rounded, or preferably a bell-shaped upper body and a flat basis, to enhance the transfer of the vibrations to the medium. This makes it easier to place the vibrating module into the sleeve as it advantageously follows the distinct shape of the sleeve. Furthermore, the filling of the sleeve can be as minimal as possible without losing its distinct shape, reducing the comfort and/or maintaining vibration transfer or comfort for the user when touching as a toy.

Fig. 6 illustrates an example of the inside of the vibrating module, in a block scheme, wherein block A represents a motor with the mechanical oscillator, block B comprises a controller and a mosfet coupling and part C comprises a battery, an on/off switch and the charging access point. The total length of the 3 blocks together is in the range of from 120 up to 150 mm, preferably of from 130 up to 140 mm. The controller is preferably a microprocessor or microcontroller, more preferably the microprocessor is an 8-bits microprocessor, or a more advanced Arduino.

The vibration module preferably further comprising an electric power source, preferably a rechargeable electric battery, optionally comprising an induction charger. It furthermore comprises preferably a bar-type vibration motor. The vibration module preferably comprises further an electric motor comprising a drive axle, and a rotor operably connected to the drive axle.

The invention is furthermore directed to a method for inducing sleepiness in a user, the method comprising: i) providing the sleep-inducing toy according to the invention in contact with the medium, and ii) activating the vibration module for a period suitable to induce and/or maintain the sleep of the user.

The invention is also directed to the use of a sleep-inducing toy according to the invention for comforting a user. The user is preferably a child which is comforted to fall asleep.

A principal object of the present invention is to provide a sleep-inducing toy that is attractive for little children as their favourite toy, handsome to carry around and that gives the comfort via both vibration and sound to let the user fall asleep easily.

From the foregoing, further variations, adaptations and modifications with respect to the sleep-inducing toy according to the present invention can be evolved by those skilled in the art to which the invention is addressed, within the scope of the following claims.

## Claims

1. A vibration module for a sleep-inducing toy, the module comprising a vibration apparatus configured to produce a mechanical vibration generating at least two mechanical waves of different frequencies for transferring via a medium to a user,
- wherein the frequencies produce both a sound and a vibration; and
- wherein the vibration module generates a mechanical oscillation wherein the mechanical vibration generates at least one frequency in the range of from 40 up to 45 Hz, and at least one frequency in the audible range, in the range of from 60 up to 65 Hz.

2. The vibration module according to claim 1, wherein the mechanical vibration generates at least one undertone in the range of from 100 up to 160 Hz, and at least one overtone with a frequency in the audible range, preferably in the range of from 200 up to 300 Hz.

3. The vibration module according to claim 1, wherein the mechanical vibration generates at least three frequencies in the range of from 40 to 45 Hz, of from 60 and up to 65 Hz, and of from 90 and up to and including 100 Hz, respectively.

4. The vibration module according to anyone of claims 1-3, further comprising a controller, wherein the vibration module is operatively connected to the controller.

5. The vibration module according to anyone of claims 1-4, further comprising an electric power source, preferably a rechargeable electric battery, optionally comprising an induction charger.

6. The vibration module according to anyone of claims 1-5, wherein the apparatus comprises a bar-type vibration motor.

7. The vibration module according to anyone of claims 1-5, wherein the apparatus comprises an electric motor comprising a drive axle, and a rotor operably connected to the drive axle.

8. The vibration module according to anyone of claims 1-7, further comprising a housing affixed to the vibration apparatus for transferring at least part of the vibrations to a sleeping pillow or a matrass in contact with the housing,
wherein the housing preferably comprises a bell-shaped upper body and a flat basis, to enhance the transfer of the vibrations to the medium.

9. A sleep-inducing toy, comprising a vibration module according to any one of claims 1 to 8, and an optionally removable outer sleeve enveloping the housing.

10. The sleep-inducing toy according to claim 9, wherein the sleeve comprises a soft, preferably washable fabric.

11. The sleep-inducing toy according to claim 9 or 10, comprising a switch, preferably in the shape of a button, for switching the controller on and off.

12. The sleep-inducing toy according to anyone of claims 9-11, having a length of from 15 centimetres to 40 centimetres, and a width of from 5 up to 20 centimetres.

13. A method for inducing sleepiness in a user, the method comprising:
i) providing the sleep-inducing toy according to anyone of claims 9-12 in contact with the medium, and
ii) activating the vibration module for a period suitable to induce and/or maintain the sleep of the user.

14. Use of a sleep-inducing toy according to anyone of claims 9-12 for comforting a user, wherein the user preferably is a child which is comforted to fall asleep.

## Patentansprüche

1. Vibrationsmodul für ein schlafinduzierendes Spielzeug, wobei das Modul eine Vibrationsvorrichtung umfasst, die so konfiguriert ist, dass sie eine mechanische Vibration erzeugt, die mindestens zwei mechanische Wellen unterschiedlicher Frequenz generiert, um sie über ein Medium an einen Benutzer zu übertragen,
- wobei die Frequenzen sowohl einen Ton als auch eine Vibration erzeugen; und
- wobei das Vibrationsmodul eine mechanische Schwingung erzeugt, wobei die mechanische Vibration mindestens eine Frequenz im Bereich von 40 bis zu 45 Hz und mindestens eine Frequenz im hörbaren Bereich im Bereich von 60 bis zu 65 Hz generiert.

2. Vibrationsmodul nach Anspruch 1, wobei die mechanische Vibration mindestens einen Unterton im Bereich von 100 bis zu 160 Hz und mindestens einen Oberton mit einer Frequenz im hörbaren Bereich, vorzugsweise im Bereich von 200 bis zu 300 Hz, generiert.

3. Vibrationsmodul nach Anspruch 1, wobei die mechanische Vibration mindestens drei Frequenzen im Bereich von 40 bis 45 Hz, von 60 und bis zu 65 Hz bzw. von 90 und bis zu und einschließlich 100 Hz generiert.

4. Vibrationsmodul nach einem der Ansprüche 1 bis 3, ferner umfassend ein Steuergerät, wobei das Vibrationsmodul funktionsmäßig mit dem Steuergerät verbunden ist.

5. Vibrationsmodul nach einem der Ansprüche 1 bis 4, ferner umfassend eine elektrische Stromquelle, vorzugsweise eine wiederaufladbare elektrische Batterie, optional umfassend ein Induktionsladegerät.

6. Vibrationsmodul nach einem der Ansprüche 1 bis 5, wobei die Vorrichtung einen stabförmigen Vibrationsmotor umfasst.

7. Vibrationsmodul nach einem der Ansprüche 1 bis 5, wobei die Vorrichtung einen Elektromotor umfasst, der eine Antriebsachse und einem Rotor umfasst, der funktionsmäßig mit der Antriebsachse verbunden ist.

8. Vibrationsmodul nach einem der Ansprüche 1 bis 7, ferner umfassend ein an der Vibrationsvorrichtung befestigtes Gehäuse zum Übertragen von zumindest einem Teil der Vibrationen auf ein Schlafkissen oder eine Matratze in Kontakt mit dem Gehäuse,
wobei das Gehäuse vorzugsweise einen glockenförmigen Oberkörper und eine flache Basis umfasst, um die Übertragung der Vibrationen auf das Medium zu verbessern.

9. Schlafinduzierendes Spielzeug, umfassend ein Vibrationsmodul nach einem der Ansprüche 1 bis 8 und eine optional abnehmbare Außenhülle, die das Gehäuse umhüllt.

10. Schlafinduzierendes Spielzeug nach Anspruch 9, wobei die Hülle einen weichen, vorzugsweise waschbaren Stoff umfasst.

11. Schlafinduzierendes Spielzeug nach Anspruch 9 oder 10, umfassend einen Schalter, vorzugsweise in Form eines Knopfes, zum Ein- und Ausschalten des Steuergeräts.

12. Schlafinduzierendes Spielzeug nach einem der Ansprüche 9 bis 11, aufweisend eine Länge von 15 bis 40 Zentimetern und eine Breite von 5 bis zu 20 Zentimetern.

13. Verfahren zum Induzieren von Schläfrigkeit bei einem Benutzer, wobei das Verfahren umfasst:
i) Bereitstellen des schlafinduzierenden Spielzeugs nach einem der Ansprüche 9 bis 12 in Kontakt mit dem Medium und
ii) Aktivieren des Vibrationsmoduls für eine Dauer, die geeignet ist, den Schlaf des Benutzers zu induzieren und/oder aufrechtzuerhalten.

14. Verwendung eines schlafinduzierenden Spielzeugs nach einem der Ansprüche 9 bis 12 zum Beruhigen eines Benutzers, wobei der Benutzer vorzugsweise ein Kind ist, das zum Einschlafen beruhigt wird.

## Revendications

1. Module de vibration destiné à un jouet induisant le sommeil, le module comprenant un appareil à vibration conçu pour produire une vibration mécanique générant au moins deux ondes mécaniques de fréquences différentes pour être transférées par l'intermédiaire d'un support à un utilisateur,
- lesdites fréquences produisant à la fois un son et une vibration ; et
- ledit module de vibration générant une oscillation mécanique, ladite vibration mécanique générant au moins une fréquence dans la plage allant de 40 à jusqu'à 45 Hz, et au moins une fréquence dans la plage audible, dans la plage allant de 60 jusqu'à 65 Hz.

2. Module de vibration selon la revendication 1, ladite vibration mécanique générant au moins une harmonique inférieure dans la plage allant de 100 jusqu'à 160 Hz, et au moins une harmonique supérieure avec une fréquence dans la plage audible, de préférence dans la plage allant de 200 jusqu'à 300 Hz.

3. Module de vibration selon la revendication 1, ladite vibration mécanique générant au moins trois fréquences dans la plage allant de 40 jusqu'à 45 Hz, allant de 60 et jusqu'à 65 Hz et allant de 90 et jusqu'à 100 Hz inclus, respectivement.

4. Module de vibration selon l'une quelconque des revendications 1 à 3, comprenant en outre un dispositif de commande, ledit module de vibration étant relié de manière fonctionnelle au dispositif de commande.

5. Module de vibration selon l'une quelconque des revendications 1 à 4, comprenant en outre une source d'énergie électrique, de préférence une batterie électrique rechargeable, comprenant éventuellement un chargeur à induction.

6. Module de vibration selon l'une quelconque des revendications 1 à 5, ledit appareil comprenant un moteur à vibrations de type barre.

7. Module de vibration selon l'une quelconque des revendications 1 à 5, ledit appareil comprenant un moteur électrique comprenant un essieu moteur et un rotor relié de manière fonctionnelle à l'essieu moteur.

8. Module de vibration selon l'une quelconque des revendications 1 à 7, comprenant en outre un boîtier fixé à l'appareil à vibration destiné à transférer au moins une partie des vibrations à un oreiller de couchage ou un matelas en contact avec le boîtier,
ledit boîtier comprenant de préférence un corps supérieur en forme de cloche et une base plate, pour améliorer le transfert des vibrations vers le support.

9. Jouet induisant le sommeil, comprenant un module de vibration selon l'une quelconque des revendications 1 à 8, et un manchon externe éventuellement amovible enveloppant le boîtier.

10. Jouet induisant le sommeil selon la revendication 9, ledit manchon comprenant un tissu doux, de préférence lavable.

11. Jouet induisant le sommeil selon la revendication 9 ou 10, comprenant un commutateur, de préférence sous la forme d'un bouton, pour allumer et éteindre le dispositif de commande.

12. Jouet induisant le sommeil selon l'une quelconque des revendications 9 à 11, comportant une longueur allant de 15 centimètres à 40 centimètres et une largeur allant de 5 jusqu'à 20 centimètres.

13. Procédé permettant d'induire la somnolence chez un utilisateur, le procédé comprenant :
i) la mise du jouet induisant le sommeil selon l'une quelconque des revendications 9 à 12 en contact avec le support, et
ii) l'activation du module de vibration pendant une durée adaptée à l'induction et/ou au maintien du sommeil de l'utilisateur.

14. Utilisation d'un jouet induisant le sommeil selon l'une quelconque des revendications 9 à 12 pour réconforter un utilisateur, ledit utilisateur étant de préférence un enfant qui est réconforté pour s'endormir.
